Europäisches Patentamt

European Patent Office

Office européen des brevets

(11) Publication number: **0 397 437**
**A1**

# EUROPEAN PATENT APPLICATION

(21) Application number: 90304927.8

(51) Int. Cl.⁵: **A61L 2/18, A61K 31/195**

(22) Date of filing: 08.05.90

(30) Priority: 11.05.89 US 350230
12.04.90 US 507061

(43) Date of publication of application:
**14.11.90 Bulletin 90/46**

(84) Designated Contracting States:
**AT BE CH DE DK ES FR GB GR IT LI LU NL SE**

(71) Applicant: **PILKINGTON VISIONCARE
HOLDINGS, INC.**
**2420 Sand Hill Road Suite 200**
**Menlo Park California 94025(US)**

(72) Inventor: **Kasper, Hans H.**
**20822 Meadow Oak Road**
**Saratoga, California 94070(US)**
Inventor: **Caldwell, Larry J.**
**4146 Cranford Circle**
**San Jose, California 95124(US)**

(74) Representative: **Pendlebury, Anthony et al**
**PAGE, WHITE & FARRER 54 Doughty Street**
**London WC1N 2LS(GB)**

(54) Method of treating human eyes and a composition thereof.

(57) An ocular lens treatment composition in aqueous form, comprising as an active ingredient an effective amount of D-penicillamine disulfide to control or substantially prevent the onset and development of cataract in the human eye.

EP 0 397 437 A1

# EP 0 397 437 A1

## METHOD OF TREATING HUMAN EYES AND A COMPOSITION THEREFOR

This invention relates to the treatment of human eyes, and more particularly, to a method of treating such eyes and a product therefor.

The ocular lens proteins have for many years been classified into the water soluble fractions and the water insoluble fractions. It is generally accepted that the newborn lens consists almost entirely of water soluble proteins and that as the lens matures and grows older, there is a slow progressive accumulation of the water insoluble protein fraction with a corresponding relative decrease in the total concentration of water soluble proteins. In the newborn rat and human lens, the soluble proteins account for more than 95% of the total protein fraction of the lens, but as the lens ages there is a progressive increase in the relative concentration of insoluble protein until it reaches a level of over 50% in the old rat lens and approximately 40% in the aged human lens.

There is also a correlation between the increase in the level of water insoluble protein and the development of presbyopia in the human lens. Presbyopia is associated with an increase in the size of the central core of the lens, the development of the so called nuclear sclerosis. Nuclear sclerosis can proceed at various rates and, in some people, results in the formation of nuclear cataracts. The human lens also develops a yellow color of increasing intensity as it ages, with the extreme being the advanced brown or black nuclear cataracts.

There is considerable interest in providing a means of preventing or delaying the onset of cataract formation in the human eye. It is believed that the onset of cataract is not simply a matter of the edge of a subject but is often related to the exposure of the eyes of the subject to U.V. light and other ionizing radiations. U.V. light and ionizing radiations are capable of generating free radicals which may initiate the formation of compounds which in turn take part in the conversion of water soluble proteins in the lens of the eye to a water insoluble form with associated formation of cataracts. The compounds which is believed are associated with the formation of water insoluble protein are fluorogens.

It has already been proposed in U.S. Patent No. 4,070,483 to utilize D.L.-penicillamine as a free radical scavenger in order to prevent or diminish the formation of fluorogen by U.V. light. This specification also discloses that the human eye contains one of the highest concentrations of glutathione in body tissues and probably acts as a free radical scavenger. Unfortunately, glutathione cannot be administered to the body to perform this scavenging function because it oxidizes rapidly. U.S. Patent No. 4,070,483 suggests the use of D.L.-penicillamine because it is a very effective free radical scavenger.

However, D.L.-penicillamine is difficult to maintain in a stable form so as to ensure its availability to the patent when required. It is desirable that it should be administered to the eye on a regular basis ideally throughout adult life so as to ensure that the maximum effort is made to avoid the onset of cataract. The material must therefore be available, off the shelf, to be either administered directly to the eye in the form of daily drops, or by incorporation in artificial lenses, which are worn on the eye.

Penicillamine unfortunately cannot be formulated for sale as an aqueous solution. Even freshly constituted solution is not stable enough to allow the preparation of multiple doses. This makes it unsatisfactory to meet the requirements of a consumer product. Penicillamine for internal use is toxic and is not administered to patients except under constant and close supervision of a physician. It is therefore undesirable and potentially unsafe to supply it for consumer use as a concentrate in a solvent, or as a solid for formulation into an aqueous solution by the end user.

We have now found that D-penicillamine disulfide (PSSP) can be formulated as an aqueous solution. Such solutions are stable at room temperature for at least 6 months. In a preferred embodiment, PSSP is formulated as an aqueous buffered isotonic (i.e., having a pH of approximately 7.2 - 7.6 -- the pH of the human eye) saline solution. When stored in plastic bottles, the aqueous buffered isotonic solution of PSSP is stable for at least two years. We have also found that despite the fact that PSSP is a more stable material, it may be acting as a free radical scavenger in the eye. In particular, we have found that it can be used in delaying or preventing the progression of cataracts in the eye.

According to the invention, there is provided an ocular lens treatment composition suitable for the control or substantial prevention of the onset and development of cataract in the human eye comprising as an active material D-penicillamine disulfide.

Our invention also includes a method in which a daily dose is administered to the eye by soaking a soft contact lens in an isotonic saline solution containing PSSP in a concentration in the range from 0.01 to 3% by weight and then placing the lens in the eye so that PSSP is delivered into the tear film in the eye over a prolonged period.

The following Examples are given as specific illustrations of the invention. It should be understood,

2

however, that the invention is not limited to the specific details set forth in the Examples.

EXAMPLE 1

A study has been carried out on rats eyes which demonstrates that PSSP is comparable in efficacy in inhibiting damage by U.V. light to the known material D-penicillamine; in particular, that both materials applied as a solution delay or inhibit the progression of cataracts in a comparable manner. In a study with pigmented rats (female, Brown-Norway) to test the efficacy of PSSP eye drops and penicillamine eye drops to prevent the onset or/and the progression of UV-B irradiation induced cataracts, the in-vivo observation period covered 62 days, the eye drops were administered in the following concentrations:

1. Penicillamine disulfide 0.3%, 1% and 3% eye drops.

2. Penicillamine 3% eye drops (freshly constituted) The animals other than a control received only daily one drop in both eyes. Those being treated were divided between those to be irradiated and those who were not selected for irradiation. Irradiation was performed every second day 4 hours after the drug application. Irradiation of some of the animals selected for irradiation was started after 1 week pretreatment with the eye drops. The results of body weight measurements and observation of general health status showed no influence of any of the treatments.

The weekly performed slit-lamp microscope examination on non-irradiated animals given drops in mydriasis showed no difference between the PSSP and the penicillamine eye drops when compared with untreated control animals.

Untreated control animals exposed to UV-B irradiation showed opacity developments in the anterior lens cortex. Treatment with 3% PSH or 3% PSSP showed a less dense opacity, and the opacity infiltration into anterior lens cortex was not as deep as in the control animals. The efficacy of the treatment to influence the progression of UV-B cataracts was better with 3% PSSP and 3% penicillamine eye drops when the eyes were pretreated with the drops for 7 days before starting UV-B irradiation.

It was also demonstrated by use of Scheimpflug photography with microdensitometric evaluation that 3% penicillamine disulfide and 3% penicillamine are able to retard cataract progression significantly. The data showed that between 2nd Scheimpflug examination in the middle of the study period and 3rd at the end, the opacities became less dense. This showed that the treatment does not only retard the cataract progression but may even support processes leading to less prominent opacities. Measurements of the light scattering intensity of the cornea showed that there is no relation of lens findings to the changes observed with corneal thickness and endothelial cell counts.

Corneal thickness and endothelial cell counts showed no general influence of the UV-B irradiation. 3% penicillamine as well as the pretreatments before irradiation with 3% penicillamine disulfide and 3% penicillamine, respectively, lower the endothelial cell density significantly and increase the corneal thickness.

The results of post-mortem lens biochemistry showed no adverse changes due to irradiation and/or treatment.

The study demonstrated clearly that at a concentration of 3% by weight there was little demonstrable difference in the performance of penicillamine and penicillamine disulfide. Lower concentrations did show an effect and are indicated as satisfactory for long term treatment of human eyes, particularly in a protective role rather than for treatment of already formed cataracts. It is unlikely that even in long term treatments, solutions with concentrations of less than 0.01 will be effective and concentrations greater than 3% are unlikely to be needed.

EXAMPLE 2

The following solutions of penicillamine and D-penicillamine disulfide were prepared (all data here expressed as percent by weight unless otherwise indicated):

3

TABLE 1

| | | COMPARATIVE EXAMPLES | | |
|---|---|---|---|---|
| | A | 1 | 2 | 3 |
| **Active Ingredient** | | | | |
| Penicillamine | - | 1.000 | 1.000 | 1.000 |
| D-penicillamine disulfide | 1.000 | | | |
| **Antioxidant***  | | | | |
| cysteine Hcl | - | - | 0.500 | - |
| dithiothretol | - | - | - | 0.500 |
| **Inactive Ingredient** | | | | |
| Edetate Disodium, USP | 0.020 | 0.020 | 0.020 | 0.020 |
| Natrosol 250H,² USP | 0.325 | 0.325 | 0.325 | 0.325 |
| Benzalkonium Chloride 10%,NF | 0.030 | 0.030 | 0.030 | 0.030 |
| Sodium Chloride, USP | 0.367 | 0.367 | 0.367 | 0.367 |
| Sodium Phosphate Dibasic Anhydrous, AR | 0.505 | 0.505 | 0.505 | 0.505 |
| Sodium Phosphate Monobasic Monohydrate | 0.061 | 0.061 | 0.061 | 0.061 |
| Purified Water, q.s. | 100 | 100 | 100 | 100 |

[1] An antioxidant was used in an attempt to stabilize penicillamine since it is believed that the degradation of penicillamine is an oxidation reaction.
[2] Trademark of Hercules, Inc. for hydroxyethylcellulose.

Samples of these solutions were placed in plastic dropper bottles and stored at 60°C., 50°C., 40°C., room temperature ("RT") and 4°C. After one, two, or four weeks some of these samples were analyzed by a high pressure liquid chromatogram ("HPLC") method with a refractive index detection for the percent of the active ingredient that remained available for treatment. The data collected are listed in Table II. The HPLC method utilized a Spectra Physics SP8800/8810 LC pump, a Micromereitics 728 auto sampler, a Shodex RI SE-61 detector, and a Zorbax QDX 4.6mm. X 35 cm. column. The mobile phase consisted of an aqueous solution of $KH_2PO_4$ (0.05 M) adjusted to pH 3 with phosphoric acid; the flow rate was 1 mL./min., and the injection volume was 20 $\mu$l. The samples were injected directly without dilution.

4

TABLE II

| Solution | Temp.(°C) | Age (weeks) | % Active Ingredient Remaining |
|----------|-----------|-------------|-------------------------------|
| A | 25 | 4 | 100 |
| A | 40 | 4 | 97.0 |
| A | 50 | 4 | 94.0 |
| A | 60 | 4 | 74.4 |
| 1 | 4 | 1 | 60.60 ± 2.97 |
| 1 | 4 | 2 | 35.00 ± 7.26 |
| 1 | RT | 1 | 49.43 ± 9.16 |
| 1 | RT | 2 | 0 |
| 1 | 40 | 2 | 0 |
| 1 | 50 | 1 | 0 |
| 1 | 60 | 1 | 0 |
| 2 | 4 | 1 | 82.65* |
| 2 | 4 | 2 | 67.00 ± 3.56 |
| 2 | RT | 1 | 64.70 ± 4.20 |
| 2 | RT | 2 | 32.70* |
| 2 | 40 | 2 | 0 |
| 2 | 50 | 1 | 0 |
| 2 | 60 | 1 | 0 |
| 3 | 4 | 1 | 83.60 ± 1.55 |
| 3 | 4 | 2 | 76.00 ± 0.82 |
| 3 | RT | 1 | 78.53 ± 1.41 |
| 3 | RT | 2 | 66.00 ± 4.32 |
| 3 | 40 | 2 | 0 |
| 3 | 50 | 1 | 0 |
| 3 | 60 | 1 | 0 |

* Average of duplicate assays.

Based on the stability data of Table II, it is highly probable that the aqueous buffered isotonic saline solution of D-penicillamine disulfide, (that is, Solution A) would be stable at room temperature for at least two years. The aqueous buffered isotonic saline solution of penicillamine is stable for no more than 4 days.

Although the invention has been described with preferred embodiments, it is to be understood that variations and modifications may be employed as will be apparent to those skilled in the art. Such variations and modifications are to be considered within the scope of the claims appended hereto.

**Claims**

1. An ocular lens treatment composition in aqueous form, comprising as an active ingredient an effective amount of D-penicillamine disulfide to control or substantially prevent the onset and development of cataract in the human eye.

2. The composition of claim 1, wherein said aqueous form is a buffered isotonic saline solution compatible with human tears.

3. A method for the treatment of the human eye, comprising administering to the eye as an active ingredient an effective amount of D-penicillamine disulfide to inhibit or reduce the rate of damage to the eye by the formation of cataracts.

4. A method of administering a daily dose of a composition as claimed in claim 1, to a human eye comprising soaking a soft contact lens before placing said lens in the eye in a solution containing D-penicillamine disulfide in a concentration in the range from 0.01 to 3% by weight and then placing the lens in the eye so that D-penicillamine disulfide is thereby released into the tear film in the eye over a prolonged period.

5. An ocular lens treatment composition in aqueous form which comprises a buffered isotonic saline solution having as an active ingredient an effective amount of D-penicillamine disulfide to control or substantially prevent the onset and development of cataract in the human eye.

6. A method for the treatment of the human eye, comprising administering to the eye, a buffered isotonic saline solution having as an active material an effective amount of D-penicillamine disulfide to inhibit or reduce the rate of damage to the eye by the formation of cataracts.

European Patent Office

**PARTIAL EUROPEAN SEARCH REPORT**
which under Rule 45 of the European Patent Convention
shall be considered, for the purposes of subsequent
proceedings, as the European search report

Application number

| DOCUMENTS CONSIDERED TO BE RELEVANT | | | EP 90304927.8 |
|---|---|---|---|

| Category | Citation of document with indication, where appropriate, of relevant passages | Relevant to claim | CLASSIFICATION OF THE APPLICATION (Int. Cl.) 5 |
|---|---|---|---|
| D,A | US - A - 4 070 483<br>(S.LERMAN)<br>* Abstract; claims 1,5,7,8 *<br>-- | 1,5 | A 61 L 2/18<br>A 61 K 31/195 |
| A | FR - A - 2 202 685<br>(SOCIETE B.F.B.)<br>* Claim 1 *<br>---- | 1,5 | |

TECHNICAL FIELDS
SEARCHED (Int. Cl.) 5

A 61 L 2/00
A 61 K 31/00
A 61 K 9/00

## INCOMPLETE SEARCH

The Search Division considers that the present European patent application does not comply with
the provisions of the European Patent Convention to such an extent that it is not possible to carry
out a meaningful search into the state of the art on the basis of some of the claims.

| | |
|---|---|
| Claims searched completely: | 1,2,5 |
| Claims searched incompletely: | - |
| Claims not searched: | 3,4,6 |
| Reason for the limitation of the search: | method for treatment of the human or animal body by therapy (see EPC Article 52(4)) |

| Place of search | Date of completion of the search | Examiner |
|---|---|---|
| VIENNA | 04-07-1990 | MAZZUCCO |

EPO Form 1505.1. 03.82